# EUROPEAN PATENT APPLICATION

(11) **EP 2 050 421 A1**
(43) Date of publication of application: **22.04.2009**
(21) Application number: 08011806.0
(22) Date of filing: 30.06.2008
(51) Int. Cl.: A61F 5/56

(54) **Apparatus for preventing sleeping respiratory obstruction and method using the same**

(30) Priority: 15.10.2007 KR 20070103357
(71) Applicant: Bio Sleep Med Co., Ltd., Seongbuk-gu Seoul (KR)
(72) Inventor: Hong, Junghwa, Anyang-si Gyeonggi-do (KR); Shin, Chol, Seoul (KR)
(74) Representative: Samson & Partner

(57) **Abstract**

An apparatus (100) for preventing a sleeping respiratory obstruction of a sleeper includes first and second air chambers (120,130) provided to correspond to both sides of a back of a human body, respectively, so as to be shrunk and expanded, an air supply unit (140) for supplying air to the first and second air chambers so as to expand the first and second air chambers, and an operation selection unit (150) for selecting whether to operate one of the first and second air chambers or to alternately operate the first and second air chambers. A controller controls (160) the air supply unit to control air supply and discharge to and from the first or second air chamber so as to operate one of the first and second air chambers or to alternately operate the first and second air chambers according to the selection result in the operation selection unit.

## Description

### Field of the Invention

The present invention relates to an apparatus for preventing a sleeping respiratory obstruction and a sleeping respiratory obstruction prevention method using the same, which are capable of allowing a sleeper to select a posture change direction suitable for preventing a sleeping respiratory obstruction and to set a time for which the changed posture is kept, thereby efficiently preventing different sleeping respiratory obstructions of individual sleepers.

### Background of the Invention

In general, a habitual snoring and an upper airway resistance syndrome classified as a sleep-disordered breathing are diseases in which the repetitive closure of the upper airway occurs during sleep. Such diseases hinder sound sleep by deteriorating sleep efficiency at night and especially decrease a blood oxygen saturation rate [see Chrokroverty S. (1994) Sleep Disorder Medicine. Butterworth-Heinemann].

The sleep-disordered breathing causes a daytime drowsiness, a deteriorated power of concentration, a memory loss, a decreased learning ability, a chronic fatigue and the like. Further, the sleeping respiratory obstruction leads to accidents at construction sites and workplaces and traffic accidents caused by a drowsy driving, thereby inflicting social and economical damages.

There have recently been several reports on a close relationship between the sleep-disordered breathing and the occurrence of obesity, high blood pressure, diabetes, dementia, cardiovascular diseases, cardiac paralysis, sexual function decline, cerebrovascular diseases, paralysis and metabolic syndrome [see Prospective study of the association between sleeping respiratory obstruction and hypertension. N Engl Med 2000; 342: 1378-1384].

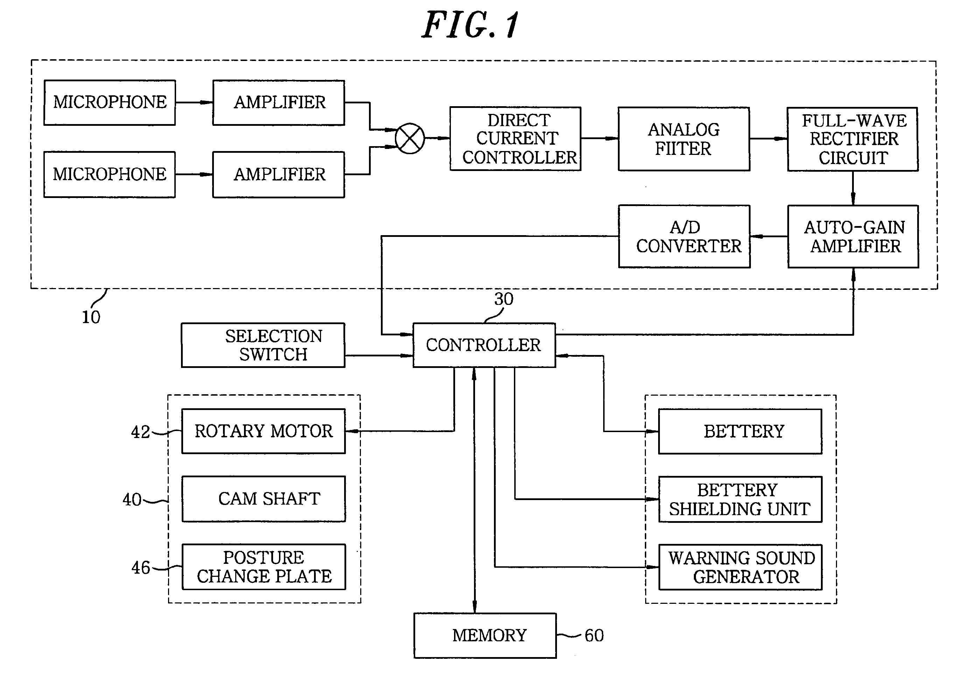

Many apparatuses and methods have been developed for solving such problems. One of the apparatuses is disclosed in a utility model No. 20-0236225 which is entitled an "APPARATUS OF DRIVING A SNORING PREVENTION PILLOW SHEET BY SENSING SNORING", and is registered in the Korean Intellectual Property Office, as shown in Fig. 1. The utility model is commonly assigned to Applicant.

The snoring prevention apparatus includes a snoring signal sensing unit 10 which inputs snoring and various other noises and outputs a desired snoring signal; a controller 30 which analyzes the signal applied from the snoring signal sensing unit 10 and starts a rotary motor driver 40 if the applied signal is confirmed as a snoring signal; a memory which stores the signal applied from the controller 30 or outputs to the controller 30; and a rotary memory driver 40 where a rotary motor 42 is driven by the signal applied from the controller 30 to drive a posture change plate 46, thereby changing the shape of a pillow sheet. The controller 30 processes the snoring signal and the noise signal applied from the snoring signal sensing unit 10 to obtain a final data and calculate a time duration when the final data continuously has a value which is larger than a threshold value. The controller 30 calculates the number of maximum points which exist for the duration if the duration is higher when compared with a set-time. The controller 30 determines the signal as the snoring signal if the number of maximum points is higher than a set value for maximum points. Further, the controller 30 applies drive signals to the rotary motor driver 40 if the snoring signal is sensed as many times as the number of the set value to drive the rotary motor 42.

In the snoring prevention apparatus according to the embodiment of the prior art, if a snoring is detected, the snoring prevention pillow sheet is automatically driven to incline the pillow sheet to one side, thereby preventing the snoring.

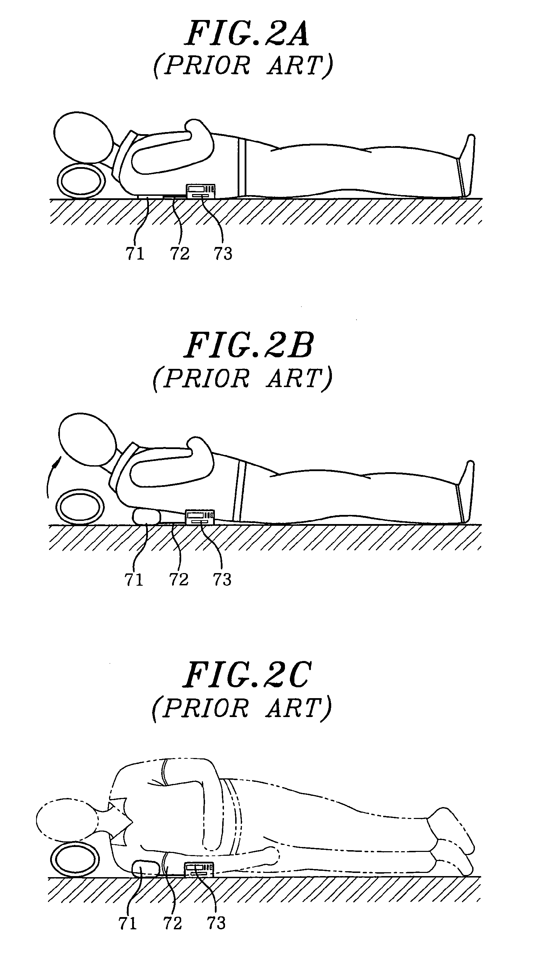

Figs. 2A to 2C are side views showing a snoring prevention apparatus according to another embodiment of the prior art. For example, a utility model No. 20-0395890, entitled "apparatus for reducing snoring," is filed for and registered in the Korean Intellectual Property Office. The snoring prevention apparatus includes an air pad 71 which detects the breath of a sleeper and is expanded by the air injected thereinto when snoring is sensed, thereby correcting the sleep posture of the sleeper; an air tube 72 which is combined with the air pad 71 to transmit the detected breathing to the next stage and to act as a passage of injecting and discharging air; a sensor for converting the breathing transmitted to the air tube 72 into an electrical signal; a preamplifier which pre-amplifies the breathing sense signal outputted from the sensor; a band pass filter unit which filters the breathing sense signal outputted from the preamplifier for each band and outputs it as a heart beat/ breathing/ snoring judging signal; a function input unit for inputting function and mode conversion signals; a central processing unit 73 which determines whether or not there is snoring based on the signal obtained by the preamplifier and the band pass filter unit in response to the signal inputted from the function input unit, which controls to display a breathing state, generates an air injection signal for expanding the air pad if it is determined as snoring, and generates a control signal to discharge the air injected into the air pad after the set time if no snoring is sensed after the air pad is expanded; a display which displays on a screen the breathing state, the heart beat status and the like of the sleeper in accordance with the state display control signal transmitted from the central processing unit 73; a driver for outputting a light emitting diode control signal, the air injection control signal and a discharge control signal, which are outputted from the central processing unit, as each corresponding drive signal for each of the air injection control and the discharge control signal; a light emitting diode (LED) for visually displaying whether or not power is supplied and a function operation state in accordance with a light emitting diode drive signal outputted from the driver; a motor for driving a pump which injects air to the air pad 71 in accordance with the air injection signal outputted from the driver; and a discharge solenoid which opens and closes a discharge valve in accordance with the discharge control signal outputted from the driver.

In another prior art snoring prevention apparatus, the air pad 71 is used to induce a posture change, thereby preventing the snoring.

However, the snoring prevention apparatus according to the embodiment of the prior art cannot return to the original state from a state in which the snoring prevention pillow sheet is inclined after detecting a snoring, unless the user manually resets the apparatus. Furthermore, the sleeper's sound sleep might be disturbed because noise is generated due to a mechanical friction between a cam shaft and a plate.

In addition, the snoring prevention apparatus according to another embodiment of the prior art is not an active type and may awake the sleeper by disturbing the sound sleep of the sleeper because it is a type which encourages to change himself/herself his/her posture. There also lies a problem in operating such an apparatus reliably because, in case that the sleeper moves away from the expansion portion of the air pad 71 when the sleeper moves or changes his position, even such a posture change cannot be induced.

Furthermore, although a direction of posture change and a time for which the changed posture is kept is different according to the sleepers, the prior art snoring prevention apparatuses do not have any unit that reflects the direction of posture change and the time for which the changed posture is kept. As a result, there is a problem in that the prior art snoring prevention apparatuses cannot efficiently prevent different sleeping respiratory obstructions of individual sleepers.

### Summary of the Invention

It is, therefore, a primary object of the invention to provide an apparatus and a method for preventing a sleeping respiratory obstruction, which are capable of unaffectedly changing a sleep posture by expanding air chambers to prevent occurrence of a sleeping respiratory obstruction and keep a sound sleep, and allowing a sleeper to select a posture change direction suitable for preventing the sleeping respiratory obstruction and to set a time for which the changed posture is kept, thereby efficiently preventing different sleeping respiratory obstructions of individual sleepers.

In accordance with an aspect of the invention, there is provided an apparatus for preventing a sleeping respiratory obstruction of a sleeper, which includes:
first and second air chambers provided to correspond to both sides of a back of a human body, respectively, so as to be shrunk and expanded;
an air supply unit for supplying air to the first and second air chambers so as to expand the first and second air chambers;
an operation selection unit for selecting whether to operate one of the first and second air chambers or to alternately operate the first and second air chambers; and
a controller for controlling the air supply unit to control air supply and discharge to and from the first or second air chamber so as to operate one of the first and second air chambers or to alternately operate the first and second air chambers according to the selection result in the operation selection unit.

In accordance with an aspect of the invention, there is provided a method for preventing a sleeping respiratory obstruction of a sleeper using first and second air chambers, which are provided to correspond to both sides of a back of a human body so as to be shrunk and expanded, the method including the steps of:
selecting whether to operate one of the first and second air chambers or to alternately operate the first and second air chambers; and
changing a posture of the sleeper by operating one of the first and second air chambers or by alternately operating the first and second air chambers according to the selection result in the step of selecting whether to operate one of the first and second air chambers or to alternately operate the first and second air chambers.

### Brief Description of the Drawings

The above and other objects and features of the present invention will become apparent from the following description of embodiments, given in conjunction with the accompanying drawings, in which:
Fig. 1 is a block diagram showing a prior art snoring prevention apparatus;
Figs. 2A to 2C are side views showing another prior art snoring prevention apparatus;
Fig. 3 is a block diagram showing the configuration of a sleeping respiratory obstruction prevention apparatus in accordance with an embodiment of the invention;
Figs. 4A and 4B are a front view and a rear view showing a human body wearable unit in a sleeping respiratory obstruction prevention apparatus in accordance with an embodiment of the invention;
Fig. 5 is a bottom view showing a mattress in a sleeping respiratory obstruction prevention apparatus in accordance with an embodiment of the invention;
Figs. 6A to 6C are flowcharts illustrating a sleeping respiratory obstruction prevention method in accordance with an embodiment of the invention;
Figs. 7A to 7C are diagrams for explaining the operation of a sleeping respiratory obstruction prevention apparatus having a vest as a human body wearable unit in accordance with an embodiment of the invention; and
Figs. 8A to 8C are diagrams for explaining the operation of a sleeping respiratory obstruction prevention apparatus having a mattress as a human body wearable unit in accordance with another embodiment of the invention.

### Detailed Description of the Embodiments

Hereinafter, embodiments of the invention will be described in detail with reference to the accompanying drawings. In the embodiments of the invention, the detailed description of known configuration or functions incorporated herein will be omitted when it may make the subject matter of the invention unclear.

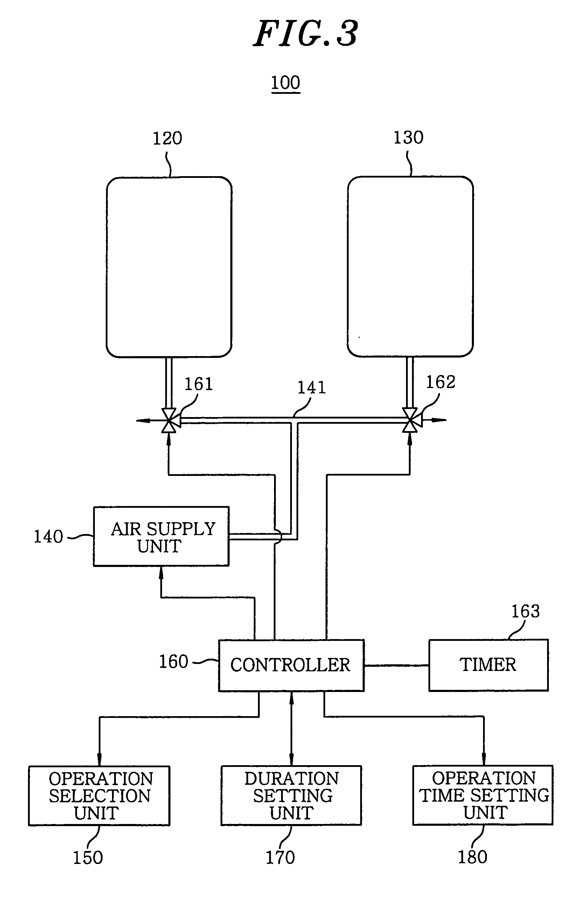

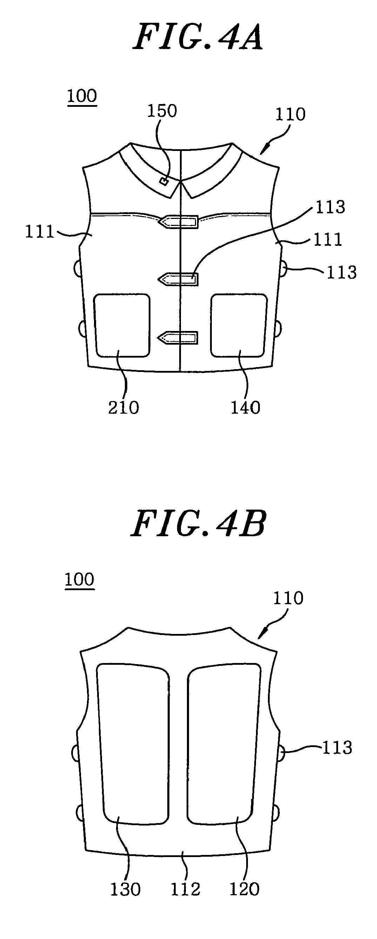

Fig. 3 is a block diagram showing the configuration of a sleeping respiratory obstruction prevention apparatus in accordance with an embodiment of the invention. Figs. 4A and 4B are a front view and a rear view showing a sleeping respiratory obstruction prevention apparatus in accordance with an embodiment of the invention. As shown in the drawings, a sleeping respiratory obstruction prevention apparatus 100 includes first and second air chambers 120 and 130 provided to correspond to both sides of a back of a human body, e.g., of a sleeper, respectively; an air supply unit 140 for supplying air to the first and second air chambers 120 and 130 so as to expand the first and second air chambers 120 and 130; an operation selection unit 150 for selecting whether to operate one of the first and second air chambers 120 and 130 or to alternately operate the first and second air chambers 120 and 130; and a controller 160 for controlling the air supply unit 140 to control air supply and discharge to and from the first and second air chambers 120 and 130 so as to operate one of the first and second air chambers 120 and 130 or to alternately operate the first and second air chambers 120 and 130 in accordance with the selection result in the operation selection unit 150.

The first and second air chambers 120 and 130 are provided to correspond to both sides of the back of the sleeper, that is, the left and right sides, respectively, so as to be shrunk and expanded.

Meanwhile, in order to prevent the first and second air chambers 120 and 130 from being misaligned due to movement and malfunctioned, the first and second air chambers 120 and 130 may be installed on a human body wearable unit 110 along with a remaining components although they are not expressly depicted, as in Fig. 4A and 4B.

The human body wearable unit 110, though not shown in drawings, may be configured in a winter jacket or a jumper form. In this embodiment, considering that it is to be worn during sleep, it may be configured in a vest shape, as shown in Figs. 4A and 4B. Furthermore, the human body wearable unit 110 may be formed in a sleeveless vest shape to make it better in permeability compared to a vest having sleeves, which in turn restrain factors increasing body temperature during sleep.

The human body wearable unit 110 may be made in various vest shapes, such as a shape of having only front adjustment, a shape of having front adjustment and side open, a shape of having neither adjustment nor open, or the like. In this embodiment, the human body wearable unit 110 is made in a shape of having front adjustment and side open. The human body wearable unit 110 includes a pair of front portions 111 which covers the upper front surface of the sleeper, and a rear portion 112 which covers the upper rear surface of the sleeper and on which the first and second air chambers 120 and 130 are provided on the left and right sides, respectively, so as to be shrunk and expanded. The front adjustment between the front portions 111 and the side open between the front portion 111 and the rear portion 112 are connected to be separable by easy-to-use fasteners 113 with which gap adjustment is easy. Here, the fastener 113 of the human wearable unit 110 is just an example, and various combining and disjoining units other than the fastener, such as zippers, rubber bands, snap buttons, and the like, may be used in order to connect the front adjustment and the side open.

Unlike this embodiment, the human body wearable unit 110 may be configured in a knapsack shape. In this case, the first and second air chambers 120 and 130 are provided on both sides of the rear surface thereof, respectively. Furthermore, shoulder belts are provided to support the knapsack on both shoulders of the sleeper, and a pelvis belt is provided to cover the pelvis below the abdomen of the sleeper. In case of the knapsack shape, the contact area to the sleeper can be minimized. In addition, when an abdominal obese person wears the human body wearable unit 110, a discomfort feeling due to pressure against the abdomen can be removed, and the sleeper can be prevented from awakening.

In both the vest shape of this embodiment and the knapsack shape, the human body wearable unit 110 to be worn by the sleeper adopts a design of a wearable unit while considering wearability and usability from a number of different design angles. Further, a functional structure and a suitable material selection are essential in such a wearable design. In particular, a material for regulating human body from harmful factors such as pleasantness, stability, electromagnetic wave shielding function, static electricity prevention function, insulation function and the like, are controlled can be selected. In order to carry out a temperature control function, the human body wearable unit 110 not only discharges the sweat excreted during sleep through the material, but is also configured to have air holes in all directions of the body or in the armpit region where sweat can come out easily, so that the heat or humidity within the clothes can be discharged quickly, thereby improving the wear comfort. And, it is necessary to consider a sewing method of enabling for minimizing the friction between the material and the human body.

The human body wearable unit 110 and particularly a portion which directly contacts to the human body may be made of health oriented materials, such as chitosan fiber, silver fiber, bamboo fiber, or the like, hi-tech materials, such as aqua-trans, cool max mesh, and the like, or environment friendly materials, such as, organic cotton, tencel, natural mineral ion health fiber, or the like. In addition, considering that the first and second air chambers 120 and 130 are expanded and shrunk, a portion that covers the first and second air chambers 120 and 130 may be made of neoprene, spandex, or the like.

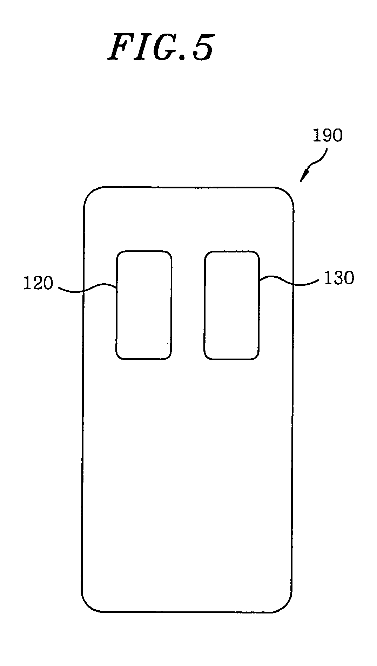

Instead of the human body wearable unit 110, as shown in Fig. 5, a mattress 190 which has the first and second air chambers 120 and 130 provided on both sides of the rear thereof may be used.

The mattress 190 provides a surface on which the sleeper lies down, and is provided with the first and second air chambers 120 and 130 in the rear surface to correspond to the back of the sleeper. With this structure, the first or second air chamber 120 or 130 is expanded upward against the rear surface to incline the mattress 190 to the right side or left side. Accordingly, the mattress 190 itself is inclined when the first or second air chamber 120 or 130 is expanded, and thus a posture change to a lateral position on a left side or right side is made accurately even if the sleeper who lies down on the mattress 190 changes his/her posture during sleep.

In order to allow the sleeper to feed soft and comfort, the mattress 190 may have a structure into which air can be injected. Alternatively, the mattress 190 may be provided with a spring therein or may be filled with foaming materials.

The first and second air chambers 120 and 130 are provided at the rear portion 112 of the human body wearable unit 110 or in the rear surface of the mattress 190 so as to be shrunk and expanded. The first and second air chambers 120 and 130 are expanded by the air supplied from the air supply unit 140, and are made in a structure or/and of a material that can be shrunk and expanded. In such a structure, the air chambers 120 and 130 may include a portion that is sheared or darted. Furthermore, for such a material, the air chambers 120 and 130 may employ, for example, synthetic resin, natural rubber, artificial rubber, fiber, and the like, which can be expanded and shrunk. The air chambers 120 and 130 can have a sufficient volume to change the posture of the sleeper, for example, from a supine position to a lateral position.

The air supply unit 140 may be provided in a pair to supply air to the first and second air chambers 120 and 130 to expand the first and second air chambers 120 and 130, respectively. In this embodiment, a single air supply unit 140 is provided so as to supply the air to the first and second air chambers 120 and 130 through branch lines of an air supply line 141. In order to supply the air to the first and second air chambers 120 and 130, a compressed gas may be used, and an air pump may be used to supply the air through pumping.

The operation selection unit 150 is provided with a plurality of buttons through which a user can select whether to operate one of the first and second air chambers 120 and 130 or to alternately operate the first and second air chambers 120 and 130. The operation selection unit 150 outputs a signal corresponding to the user' s selection to the controller 160.

The controller 160 controls the air supply unit 140 so as to operate one of the first and second air chambers 120 and 130 or to alternately operate the first and second air chambers 120 and 130 in accordance with the selection result in the operation selection unit 150, and also controls air supply and discharge to and from the first or second air chamber 120 or 130. Then, the posture of the sleeper is changed, for example, from the supine position to the lateral position of the left side or right side, thereby opening the upper airway of the sleeper, which in turn corrects the disordered breathing of the sleeper.

The controller 160 can control the air supply unit 140 to supply the air to the first or second air chamber 120 or 130 for expansion thereof and to discharge the air from the first or second air chamber 120 or 130 for shrinkage thereof. For example, if the air supply unit 140 is an air pump, the air supply and discharge are achieved through a forward rotation and a reverse rotation. In addition, although it is not limited thereto, three-way valves 161 and 162, as in this embodiment, are controlled by the controller 160 and installed on the branch lines of the air supply line 141 which are connected to the first and second air chambers 120 and 130. Through the operations of the three-way valves 161 and 162, the air supplied from the air supply unit 140 is selectively supplied to the first or second air chamber 120 or 130. Furthermore, the first or second air chamber 120 or 130 is pressurized by the weight of the sleeper, so that the air in the first or second air chamber 120 or 130 is discharged for the shrinkage thereof. The three-way valves 161 and 162 may include solenoid valves.

In the present invention, a duration setting unit 170 may be further provided to set a duration for which the first or second air chamber 120 or 130 is kept expanded. Accordingly, the controller 160 expands the first or second air chamber 120 or 130 for the duration set by the duration setting unit 170, for example, 15, 30, 45, or 60 minutes. Therefore, when the duration set by the duration setting unit 170 elapses after the first or second air chamber 120 or 130 is expanded, the three-way valve 161 or 162 is controlled such that the air is discharged from the first or second air chamber 120 or 130.

An operation time setting unit 180 may be further provided to set an operation time at which the first or second air chamber 120 or 130 will start to operate from the beginning, preferably, after the apparatus is turned on, for example, at which the sleeper gets to sleep and undergoes a sleeping respiratory obstruction after turning on the apparatus 100. Accordingly, when the operation time set by the operation time setting unit 180 reaches, the controller 160 controls the air supply unit 140 and the three-way valve 161 or 162 to operate the first or second air chamber 120 or 130 for the expansion or shrinkage thereof.

Meanwhile, the controller 160 communicates with a timer 163 that counts the duration set by the duration setting unit 170 or the operation time set by the operation time setting unit 180, and outputs a signal when the duration elapses or the operation time reaches. With the timer 163, the controller 160 can determine whether or not the duration set by the duration setting unit 170 elapses or whether or not the operation time set by the operation time setting unit 180 reaches.

The operation of the sleeping respiratory obstruction prevention apparatus 100 made in such a structure will be superseded with the detail description of the sleeping respiratory obstruction prevention method in accordance with the invention.

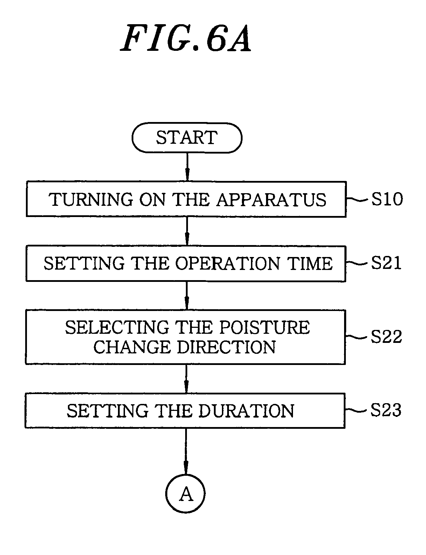

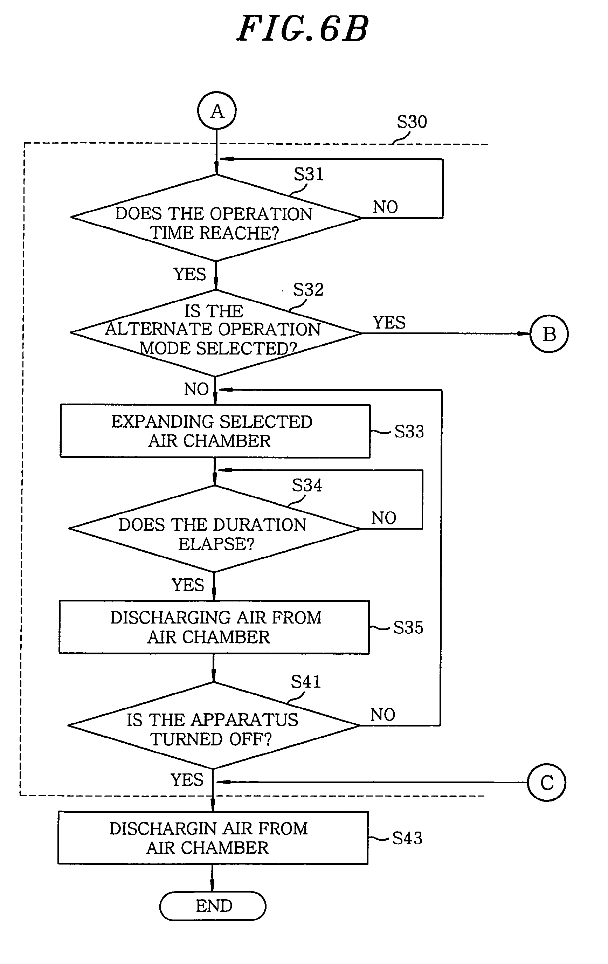

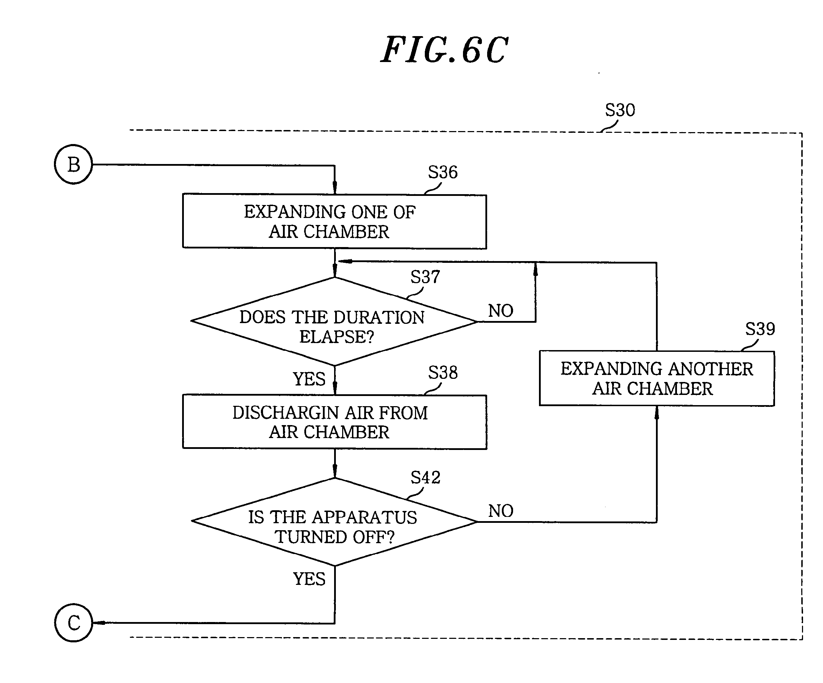

Figs. 6A to 6C are flowcharts illustrating a sleeping respiratory obstruction prevention method. As shown in these drawings, the sleeping respiratory obstruction prevention method includes the steps of: selecting a posture change direction of the sleeper in step S22; and changing a posture of the sleeper by operating the first or second air chamber 120 or 130 according to the selection result in the step of selecting the posture change direction in step S30.

The step S22 is to select whether to operate one of the first and second air chambers 120 and 130 or to alternately operate the first and second air chambers 120 and 130. The user selects one of a mode to operate the first air chamber 120, a mode to operate the second air chamber 130, and a mode to alternately operate the first and second air chambers 120 and 130 through the operation selection unit 150. In this way, a mode suitable for preventing the sleeping respiratory obstruction of the sleeper is selected.

Meanwhile, the sleeping respiratory obstruction prevention method may further include the steps of: setting an operation time at which the first or second air chamber 120 or 130 will start to operate from the beginning in step S21; and setting a duration for which the first or second air chamber 120 or 130 is kept expanded in step S23. In this embodiment, the step S21 and the step S23 are provided before and after the step S22, respectively, but the invention is not limited thereto, and the sequence may be changed.

The step S21 is to set an operation time, at which the first or second air chamber 120 or 130 will start to operate from the beginning, for example, after the apparatus 100 is turned on in step S10, through the use of the operation time setting unit 180. For example, the operation time may be set to a time at which the sleeper gets to sleep and undergoes the sleeping respiratory obstruction after turning on the apparatus 100. The operation time may be set to different values according to individual sleepers.

The step S23 is to set a duration, for which the first or second air chamber 120 or 130 is kept expanded when the first or second air chamber 120 or 130 is repeated expanded and shrunk, by use of the duration setting unit 170. The duration may be set such that the sleeping respiratory obstruction of the sleeper can be effectively prevented. The duration may be set in a range of, for example, 15 to 60 minutes. For example, the duration may be set to one of 15, 30, 45, or 60 minutes.

The sleeper turns on the apparatus 100 before he/she wears the human body wearable unit 110 or lies down on the mattress 190 in step S10. However, the invention is not limited thereto. For example, in step S10, the sleeper may turn on the apparatus 100 in a state where he/she wears the human body wearable unit 110 or lies down on the mattress 190.

The step S30 is to change the posture of the sleeper by operating one of the first and second air chambers 120 and 130 or alternately operating the first and second air chambers 120 and 130 according to the selection result in the step S22. Hereinafter, the step S30 of selecting the posture change direction will be described in detail.

First, it is determined in step S31 whether or not the operation time set in the step S21 reaches through counting of the timer 163. If the operation time reaches, the controller 160 controls the air supply unit 140 and the three-way valve 161 or 162 to operate first or second air chamber 120 or 130.

Thereafter, it is determined in step S32 whether or not the mode to alternately operate the first and second air chambers 120 and 130 is selected in the step S22. If, in step S32, the mode to operate one of the first and second air chambers 120 and 130 is selected, not the alternate operation mode, a control process advances to step S33 where the controller 160 expands the first or second air chamber 120 or 130 according to the selection result in the step S22.

Next, it is determined in step S34 whether or not the duration set in the step S23 elapses after the first or second air chamber 120 or 130 is expanded. If the duration elapses, a control process goes to step S35 where the controller 160 controls the three-way valve 161 or 162 such that the air is discharged from the expanded first or second air chamber 120 or 130. In this way, the selected first or second air chamber 120 or 130 is only expanded for the duration set in the step S23.

Subsequently, it is determined in step S41 whether or not the apparatus 100 is turned off. If the determination is negative, a control process goes to the step S33 to repeat the above steps S33, S34, and S35. In this way, the selected first or second air chamber 120 or 130 is repeatedly shrunk and expanded.

However, in the step S32, if it is determined that the mode to alternately operate the first and second air chambers 120 and 130 is selected, a control process flows to step S36 where the controller 160 controls the air supply unit 140 and the three-way valve 161 or 162 to expand one of the first and second air chambers 120 and 130.

Next, when the air chamber is fully expanded, it is determined in step S37 whether or not the duration set in the step S23 elapses. If the duration elapses, a control process goes to step S38 where the air is discharged from the expanded air chamber.

Thereafter, in step S42, it is determined whether or not the apparatus 100 is turned off. If the determination is negative, a control process to step S39 where another air chamber is expanded.

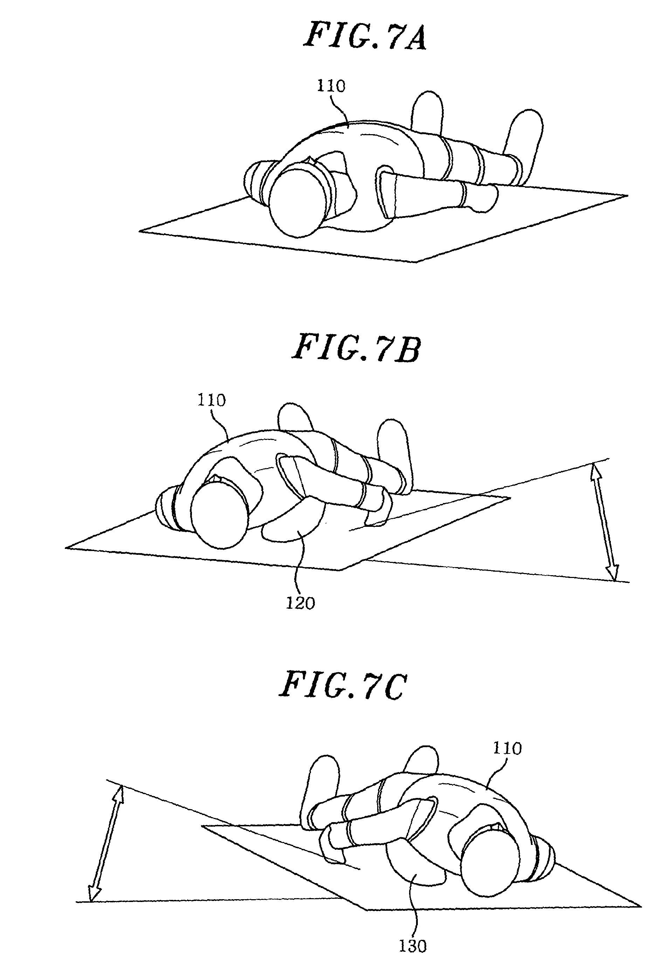

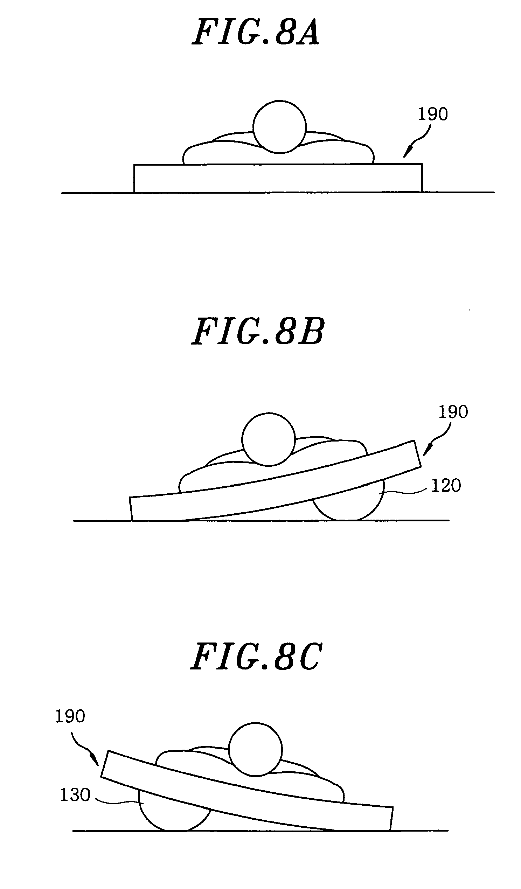

Subsequently, a control process returns to the step S37 to repeatedly perform the steps S37 and S38 until the apparatus 100 is turned off. In this way, as shown in Figs.7A to 7C and 8A to 8C, the first and second air chambers 120 and 130 are alternately expanded and shrunk.

Next, in either of steps S41 and S42, if it is determined that the apparatus is turned off, the air is discharged from the first or second air chamber 120 or 130 for a predetermined time. Here, the predetermined time is proportional to the size of the first or second air chamber 120 or 130. The predetermined time may be set to a time for which the first or second air chamber 120 or 130 sufficiently discharges the air therein.

According to the sleeping respiratory obstruction prevention apparatus and the sleeping respiratory obstruction prevention method using the same of the invention, the air chambers provided to correspond to the back of the sleeper are expanded to unaffectedly change the sleep posture so as to stop occurrence of a sleeping respiratory obstruction and to keep a sound sleep. Furthermore, since the air chambers are accurately positioned at the back of the sleeper, a posture change for preventing the sleeping respiratory obstruction can be made accurately even if the air chambers are expanded and the position and posture of the sleeper are changed during sleep.

In addition, the sleeper is allowed to select the posture change direction suitable for preventing the sleeping respiratory obstruction and to set the time for which the changed posture is kept. Therefore, different sleeping respiratory obstructions of individual sleepers can be efficiently prevented. While the invention has been shown and described with respect to the preferred embodiments, it will be understood by those skilled in the art that various changes and modification may be made without departing from the scope of the invention as defined in the following claims.

## Claims

1. An apparatus for preventing a sleeping respiratory obstruction of a sleeper, the apparatus comprising:
first and second air chambers provided to correspond to both sides of a back of a human body, respectively, so as to be shrunk and expanded;
an air supply unit for supplying air to the first and second air chambers so as to expand the first and second air chambers;
an operation selection unit for selecting whether to operate one of the first and second air chambers or to alternately operate the first and second air chambers; and
a controller for controlling the air supply unit to control air supply and discharge to and from the first or second air chamber so as to operate one of the first and second air chambers or to alternately operate the first and second air chambers according to the selection result in the operation selection unit.

2. The apparatus of claim 1, further comprising:
a human body wearable unit to be worn by the sleeper and provided with the first and second air chambers at a rear surface thereof.

3. The apparatus of claim 1, further comprising:
a mattress provided with the first and second air chambers on both sides of a rear surface thereof.

4. The apparatus of claim 1, further comprising:
a duration setting unit for setting a duration in which the first or second air chamber is kept expanded,
wherein the controller controls to expand the first or second air chamber for the duration set by the duration setting unit.

5. The apparatus of claim 1, further comprising:
an operation time setting unit for setting an operation time at which the first or second air chamber will start to operate from the beginning,
wherein the controller control to operate the first or second air chamber when the operation time set by the operation time setting unit reaches.

6. A method for preventing a sleeping respiratory obstruction of a sleeper using first and second air chambers, which are provided to correspond to both sides of a back of a human body so as to be shrunk and expanded, the method comprising the steps of:
selecting whether to operate one of the first and second air chambers or to alternately operate the first and second air chambers; and
changing a posture of the sleeper by operating one of the first and second air chambers or by alternately operating the first and second air chambers according to the selection result in the step of selecting whether to operate one of the first and second air chambers or to alternately operate the first and second air chambers.

7. The method of claim 6, further comprising the step of:
setting a duration in which the first or second air chamber is kept expanded,
wherein the step of changing the posture expands the first or second air chamber for the duration set in the step of setting the duration.

8. The method of claim 6, further comprising the step of:
setting an operation time at which the first or second air chamber will start to operate from the beginning,
wherein the step of changing the posture operates the first or second air chamber when the operation time set in the step of setting the operation time reaches.
